# EUROPEAN PATENT APPLICATION

(11) **EP 1 798 233 A1**
(43) Date of publication of application: **20.06.2007**
(21) Application number: 05292739.9
(22) Date of filing: 19.12.2005
(51) Int. Cl.: C07D 487/04, A61K 31/498, A61P 9/00

(54) **Pyrrolo[1,2-a]quinoxaline derivatives as Adenosine A3 receptor modulators and uses thereof**

(71) Applicant: Faust Pharmaceuticals, 67400 Illkirch (FR)
(72) Inventor: Schann, Stephan, 67118 Geispolsheim (FR); Mayer, Stanislas, 67114 Eschau (FR); Gardan, Sophie, 67100 Strasbourg (FR)

(57) **Abstract**

The present invention provides new compounds of formula (I) displaying high affinity for adenosine A₃ receptors. It also provides modulators of adenosine A₃ receptors. It further provides compounds for the treatment and/or prophylaxis of conditions and diseases where adenosine A₃ receptor plays a role. Pharmaceutical compositions for the treatment and/or prophylaxis of conditions and diseases where adenosine A₃ receptors play a role are also described.

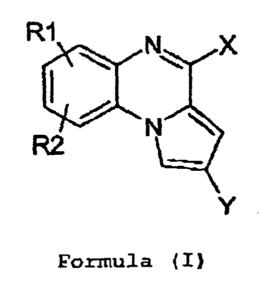

## Description

The present invention provides new compounds displaying high affinity for adenosine A₃ receptors. It also provides modulators of adenosine A₃ receptors. It further provides compounds for the treatment and/or prophylaxis of conditions and diseases where adenosine A₃ receptor plays a role. Pharmaceutical compositions for the treatment and/or prophylaxis of conditions and diseases where adenosine A₃ receptors play a role are also described.

Adenosine is an ubiquitous modulator of numerous physiological activities, particularly within the cardiovascular and nervous systems. The effects of adenosine are mediated by specific cell surface receptor proteins. Adenosine modulates diverse physiological functions including induction of sedation, vasodilatation, suppression of cardiac rate and contractility, inhibition of platelet aggregability, stimulation of gluconeogenesis and inhibition of lipolysis. In addition to its effects on adenylate cyclase, adenosine has been shown to open potassium channels, reduce flux through calcium channels, and inhibit or stimulate phosphoinositide turnover through receptor-mediated mechanisms (Muller C.E. and Stein B., Current Pharmaceutical Design, 2:501, 1996*,* and Muller C.E., Exp. Opin. Ther. Patents, 7(5):419, 1997*).*

Adenosine receptors belong to the superfamily of purine receptors. Four major classes of adenosine receptors have been pharmacologically, structurally and functionally characterized ( Fredholm et al., Pharm. Rev. (1994) 46:143-156*)* and classified A₁, A₂ₐ, A_{2B} and A₃ (referred to as A₁, A_{2Λ}, A_{2B} and A₃ receptors among the present application). A₁ receptors are coupled to the inhibition of adenylate cyclase through Gᵢ proteins and have also been shown to couple to other second messengers systems, including inhibition or stimulation of phosphoinositol turnover and activation of ion channels. A_{2A} and A_{2B} receptors are coupled to Gₛ proteins promoting the activation of adenylate cyclase, and leading to an increase in cellular cAMP levels. The A₁, A₂ₐ and A_{2B} subtypes were initially discovered by a study of agonist pharmacology but the A₃ subtype was recently discovered by molecular biology studies. In fact, the A₃ receptor sequence was first identified in a rat testes cDNA library, and this sequence was later cloned by homology to other G-protein coupled receptors (GPCRs) from a rat brain cDNA library.

In rat, the transcript of the A₃ receptor is found primarily in the central nervous system, testes, lung, kidneys, heart and inflammatory/immune cells. It seems to be a large interspecies difference in peripheral distribution of A₃ receptor among mammals: in the sheep, the transcript is especially found in the lung, spleen, pars tuberalis, pineal gland and inflammatory/immune cells, with lower levels in testis, kidneys and brain (Linden et al., Mol. Pharmacol. (1993), 44:524-532*);* the human transcript is widespread and the most abundant expression is detected in the lung and liver (Salvatore et al., Proc. Natl. Acad. Sci . USA (1993), 90:10365-10369*).* A₃ receptors have been shown to be involved in the pathophysiology of asthma and additional inflammatory conditions as chronic obstructive pulmonary disease (Meade C. J. et al., Life Science (2001), 69, 1225-1240*;* Polosa et al., Eur Respir. (2002), 20:488-496*;* Cronstein B.N. et al., Arthritis Rheum. (1995), 38, 1040-1045*).* A₃ receptors are also involved in the regulation of intraocular pressure (Yang et al., Current Eye Research 30 (2005), 747-754) and modulating them is a novel approach for treating glaucoma.
Further investigations have identified A₃ receptors in a number of human cancers, including pancreatic cancer, colon cancer, breast cancer, lung cancer and human malignant melanoma. Surprisingly, A₃ receptors are found at higher concentrations in the cancerous cells as compared to normal healthy tissue. In the patent US 10/134,219 (Baraldi et al.) which is hereby incorporated by reference, the inventors demonstrated success in using A₃ receptor antagonists to induce apoptosis in human cancers. It shows that the use of A₃ antagonists allows targeting of the cancer cells for apoptosis, thereby reducing anticipated side effects in treatment of patients.

To date, IB-MECA (N⁶-(3-iodobenzyl)-adenosine-5'-N-methyluronamide) and Cl-IB-MECA (2-chloro-N⁶(3-iodobenzyl)-adenosine-5*'*-N-methyluronamide) (Jacobson et al., FEBS, 336 (1), 57-60*;* Kim H. O. et al., Journal of Medicinal Chemistry, 1994, 37, 3614-3621*)* are the most potent and specific A₃ receptors agonists and have been widely used in a variety of studies. To show the interest of an A₃ receptor agonist an example is the prevention of ischemia. In fact, adenosine is released in large amounts during myocardial ischemia and can mediate potentially important protective functions in the cardiovascular system. Therefore, specific A₃ agonists can precondition the heart when given before the onset of ischemia and can cause a reduction of the consequences of the onset (Liang et al., patent US 6,211,165). Agonists selective for the A₃ receptor are of interest as cerebroprotective (Jacobson et al., Trends Pharmacol. Sci. (1996), 17, 108-113*),* cardioprotective (Nakano et al., Pharmacol. Ther. (2000) 86, 263-275 and Dougherty et al., FASEB J. (1998) 12, 1785-1792*)* and anticancer agents (Fishman et al., Oncogene (2002), 21, 4060-4064*).*

Mast cell degranulation is a component of myocardial reperfusion injury, hypersensitivity reactions (asthma, allergic rhinitis, and urticaria), ischemic bowel disease, autoimmune inflammation, and atopic dermatitis. Selective A₃ receptor antagonists can be used to treat and/or prevent these diseases and generally the pathologic effects that result from mast cell degranulation. Specific A₃ receptor antagonists already identified *(*Jacobson et al., US 6,376,521) are currently developed in several applications among which asthma, chronic obstructive pulmonary disease, glaucoma and other intraocular pressure troubles (Okamura et al., Bioorganic & Med Chem Letters, 14 (2004), 3775-3779*).* To date, A₃ receptor antagonists are sought to be useful as antiasthmatic, antidepressant, antiarrhythmic, renal protective, antiparkinson and cognitive enhancing drugs, but also as anti-inflammatory or possibly anti-ischemic agents in the brain.

Additional adenosine receptor modulators are needed as pharmacological tools and are of considerable interest as drugs for the treatment and/or prophylaxis of various diseases where A₃ receptors play a role. There remains a need for specific modulators of A₃ receptors. The present invention provides compounds with improved potency for A₃ receptor binding affinity and A₃ selectivity against other subtypes. The invention provides also methods of using these compounds to selectively modulate A₃ receptors in patients in need thereof, and pharmaceutical compositions comprising such compounds. These and other objects and advantages of the present invention, as well as additional inventive features, will be apparent from the description of the invention provided herein.

The present invention provides compounds of formula (I) : 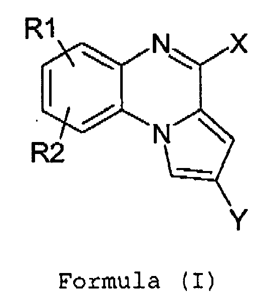 wherein:
**R1** and **R2** are independently from each other hydrogen, halogen, CN, CF₃, OCF₃, lower alkyl, COOH, O-(lower alkyl), S-(lower alkyl), N-(lower alkyl) (lower alkyl), heterocycloalkyl, aryl or heteroaryl;
**X** represents **R3** or CO**R3,** O-**R3** or S-**R3,** N-**R3R4,** NHCO**R3,** N (lower alkyl) CO**R3**, NHSO₂**R3**, N (lower alkyl) SO₂**R3** or NHCONH**R3** with
   **R3** and **R4** are independently from the other hydrogen, alkyl, CF₃, aryl, heteroaryl, alkylaryl or alkylheteroaryl;
**Y** represents a **(A)ₙ-B** group wherein
   **n** represents either 0 or 1,
   **A** is a moiety selected in the group consisting of O, S, NH, N-(lower alkyl), N-aryl, N-heteroaryl,
   **B** is an aryl or a heteroaryl group;
as well as pharmaceutically acceptable salt thereof, a prodrug of the compound or the salt, or a solvate or hydrate of the compound, the salt or the prodrug.

In a further embodiment, the invention provides compounds of formula (II) as follows: 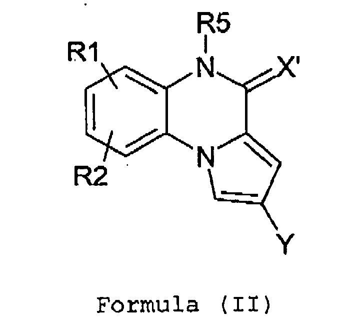 wherein:
**R1, R2** and **Y** are as defined in formula (I);
**X'** is selected from O or S
**R5** is selected from alkyl, alkylaryl or alkylheteroaryl.

As used herein, the term **"alkyl"** includes straight or branched chain saturated hydrocarbon residues with 1-8 carbon atoms, such as methyl, ethyl, n-propyl, isopropyl, n-butyl, i-butyl, s-butyl and t-butyl. These alkyls can be further substituted with groups such as COOH, heterocycloalkyl, CF₃, OH, O-(lower alkyl) or N-(lower alkyl)(lower alkyl).
As used herein, the term **"lower alkyl"** includes straight or branched chain saturated hydrocarbon residues with 1-4 carbon atoms, such as methyl, ethyl, n-propyl, isopropyl, n-butyl, i-butyl, s-butyl or t-butyl.
The term **"aryl"** refers to a 6-10 atoms aromatic hydrocarbon ring or fused aromatic hydrocarbon ring system containing at least one unsaturated aromatic ring. Examples of the term "aryl" are phenyl, naphthyl and 1,2,3,4-tetxahydronaphthyl. These aryls can be further substituted with groups such as halogen, CN, CF₃, OCF₃, lower alkyl, COOH, O-(lower alkyl), S-(lower alkyl), N-(lower alkyl)(lower alkyl) or heterocycloalkyl.
The term **"heteroaryl"** means a 5-10 atoms aromatic ring or fused aromatic rings containing one or more O, S, or N atoms. Examples of heteroaryls include pyridinyl, quinolinyl, isoquinolinyl, pyridazinyl, pyrimidinyl, pyrazinyl, furyl, benzofuryl, thienyl, benzothienyl, pyrrolyl, indolyl, pyrazolyl, indazolyl, oxazolyl, benzoxazolyl, thiazolyl, benzothiazolyl, imidazolyl, benzimidazolyl, and tetrazolyl. These heteroaryls can be further substituted with groups such as halogen, CN, CF₃, OCF₃, lower alkyl, COOH, O-(lower alkyl), S-(lower alkyl), N-(lower alkyl)(lower alkyl) or heterocycloalkyl.

The term **"heterocycloalkyl"** means a 4-7 atoms ring containing one or more O, S, or N atoms. Examples of heterocycloalkyls include azetidinyl, pyrrolidinyl, tetrahydrofuranyl, imidazolinyl, pyrolidin-2-one, morpholinyl, thiomorpholinyl, piperidinyl, piperidin-2-one, piperazinyl, N-alkyl-piperazinyl.
The term **"alkylaryl"** means an alkyl-aryl-group or radical
wherein alkyl and aryl have the meanings as defined above. Illustrative examples of an alkylaryl group or radical include benzyl, 2-phenylethyl, 3-phenylpropyl, 4-phenylbutyl, 3-methyl-3-phenylpropyl, 1-naphthylmethyl, 1-naphthylethyl.
The term **"alkylheteroaryl"** means an alkyl-heteroaryl-group or radical wherein alkyl and heteroaryl have the meanings as defined above.
The term **"halogen"** refers to bromine, chlorine, fluorine, or iodine. Pharmaceutically acceptable salts of compounds of formula (I) include salts with inorganic or organic acids, e.g. hydrochloric, hydrobromic, nitric, carbonic, formic, monohydrogencarbonic, phosphoric, monohydrogenphosphoric, dihydrogenphosphoric, perchloric, sulfuric, monohydrogensulfuric, hydroiodic, phosphorous, acetic, lactic, propionic, butyric, isobutyric, palmoic, maleic, glutamic, hydroxymaleic, malonic, benzoic, succinic, glycolic, suberic, fumaric, mandelic, phthalic, salicylic, benzenesulfonic, p-tolylsulfonic, citric, tartaric, methanesulfonic and hydroxynaphthoic acids. Pharmaceutically acceptable salts of compounds of formula (I) can also include salts with inorganic bases, e.g. alkali metal bases, especially sodium or potassium bases or alkaline-earth metal bases, especially calcium or magnesium bases, or with pharmaceutically acceptable organic bases.

The invention is based, at least in part, on the discovery that the compound described above can be used to prevent and/or treat various medical disorders and/or conditions where A₃ receptors play a role. These disorders and/or conditions are associated with, for example, asthma, hypersensitivity, rhinitis, hay fever, serum sickness, allergic vasculitis, atopic dermantitis, dermantitis, psorasis, eczema, idiopathic pulmonary fibrosis, eosinophillic chlorecystitis, chronic airway inflammation, chronic obstructive pulmonary disease, hypereosinophilic syndromes, eosinophilic gastroenteritis, edema, urticaria, eosinophilic myocardial disease, episodic angioedema with eosinophilia, inflammatory bowel disease, ulcerative colitis, allergic granulomatosis, carcinomatosis, eosinophilic granuloma, familial histiocytosis, hypertension, mast cell degranulation, tumor, cardiac hypoxia, cerebral ischemia, diuresis, renal failure, neurological disorder, mental disorder, cognitive disorder, myocardial ischemia, bronchoconstriction, arthritis, autoimmune disease, Crohn's disease, Grave's disease, diabetes, multiple sclerosis, anaemia, psoriasis, fertility disorders, lupus erthyematosus, reperfusion injury, brain arteriole diameter, the release of allergic mediators, scleroderma, stroke, global ischemia, central nervous system disorder, cardiovascular disorder, renal disorder, inflammatory disorder, gastrointestinal disorder, eye disorder, allergic disorder, respiratory disorder, or immunological disorder.

Among preferred compounds of formula (I), preferred embodiments include those wherein **R1, R2** and **X** are as defined in formula (I) and **Y** represents an aryl group.

Further preferred embodiments include compounds of formula (I) wherein **R1** is hydrogen, **R2** and **X** is as defined in formula (I) and **Y** represents an aryl group.

Other preferred compounds of formula (I) are those wherein **R1** and **R2** are hydrogen, **X** is as defined in formula (I) and **Y** represents an aryl group.

Additional **preferred** compounds of formula (I) are those wherein **R1** and **R2** are hydrogen, **X** represents **R3,** O**R3** or S-**R3**, N-**R3R4**, with:
**R3** and **R4** are independently from the other hydrogen, alkyl, CF₃, aryl, heteroaryl, alkylaryl or alkylheteroaryl;
and **Y** represents an aryl group.

The compounds of general formula (I) and their pharmaceutically acceptable salts can be manufactured according to methods described in the following schemes:

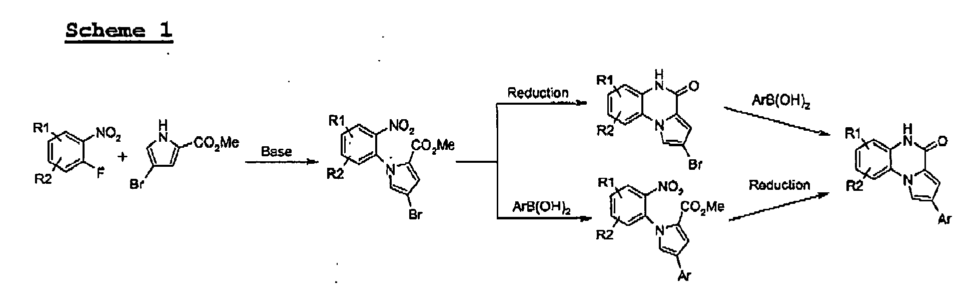

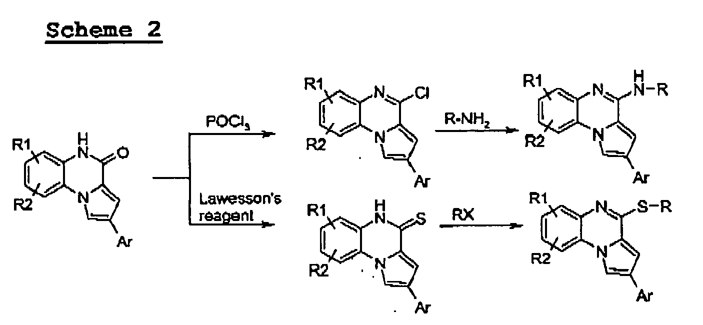

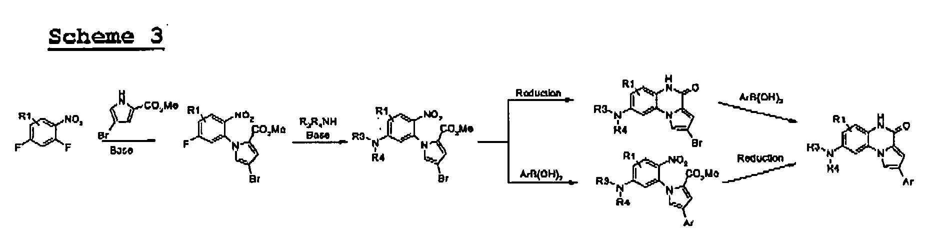

The present invention pertains to methods for modulating A₃ receptor functioning by the administration of a therapeutically effective amount of a compound of general formula (I) to a patient in need thereof, such that modulation of the adenosine receptor's activity occurs.
The modulation of the adenosine receptor's activity can be assessed in binding assays which are well known to one skilled in the art. The following methods are widely used: for A₁ receptor the procedure described by Townsend-Nicholson and Schofield (J. Biol. Chem. (1994), 269:2373-2376), for A_{2A} receptor the procedure described by Luthin et al. (Mol. Pharmacol. (1995), 47:307-313), for A_{2B} receptor the procedure described by Stehle et al. (Mol. Endocrinol. (1992), 6:384-393), and for A₃ receptor the procedure described by Salvatore et al. (Proc. Natl. Acad. Sci. (1993), 90:10365-10369).

In a preferred embodiment, the compound of formula (I) is an A₃ receptor antagonist.

In a further embodiment, the invention provides methods of selectively blocking A₃ receptor in a mammal by administration of a therapeutically effective amount of a compound of formula (I) to a patient in need thereof, such that blockage of the adenosine receptor's activity occurs.

Preferred compounds of formula (I) according to the invention are:
- *2-Phenyl-5H-pyrrolo[1,2-a]quinoxalin-4-one* 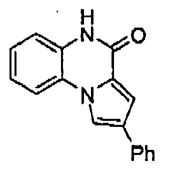
- *2-(4-Methoxy-phenyl)-5H-pyrrolo[1,2-a]quinoxalin-4-one.*

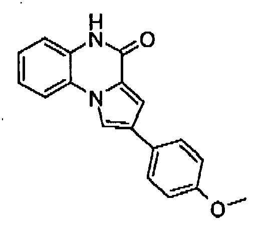

The invention provides also the use of a compound of general formula (I) in the manufacture of a drug for use in the treatment and/or prophylaxis of conditions and/or disorders where A₃ receptors play a role.

The present invention further provides a pharmaceutical composition comprising a pharmaceutically effective amount of a compound of the formula (I), or pharmaceutically acceptable salts thereof, in combination with a pharmaceutically acceptable carrier, diluent or excipient.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality of life.

In another embodiment, the invention provides a pharmaceutical composition for the treatment and/or prophylaxis of a disorders and/or or condition where A₃ receptors play a role, associated with, for example, asthma, hypersensitivity, rhinitis, hay fever, serum sickness, allergic vasculitis, atopic dermantitis, dermantitis, psorasis, eczema, idiopathic pulmonary fibrosis, eosinophillic chlorecystitis, chronic airway inflammation, chronic obstructive pulmonary disease, hypereosinophilic syndromes, eosinophilic gastroenteritis, edema, urticaria, eosinophilic myocardial disease, episodic angioedema with eosinophilia, inflammatory bowel disease, ulcerative colitis, allergic granulomatosis, carcinomatosis, eosinophilic granuloma, familial histiocytosis, hypertension, mast cell degranulation, tumor, cardiac hypoxia, cerebral ischemia, diuresis, renal failure, neurological disorder, mental disorder, cognitive disorder, myocardial ischemia, bronchoconstriction, arthritis, autoimmune disease, Crohn's disease, Grave's disease, diabetes, multiple sclerosis, anaemia, psoriasis, fertility disorders, lupus erthyematosus, reperfusion injury, brain arteriole diameter, the release of allergic mediators, scleroderma, stroke, global ischemia, central nervous system disorder, cardiovascular disorder, renal disorder, inflammatory disorder, gastrointestinal disorder, eye disorder, allergic disorder, respiratory disorder, or immunological disorder.

The compounds of the invention may be administered in a form suitable for oral use, for example a tablet, capsule, aqueous or oily solution, suspension or emulsion; for topical use including transmucosal and transdermal use, for example a cream, ointment, gel, aqueous solution or suspension, salve, patch or plaster; for nasal use for example a snuff, nasal spray or nasal drops; for vaginal or rectal use, for example a suppository; for administration by inhalation, for example a finely divided powder or a liquid aerosol; for sub-lingual or buccal use, for example a tablet or a capsule; or for parenteral use (including intravenous, subcutaneous, intramuscular, intravascular or infusion), for example a sterile aqueous solution or suspension, or depot injection formulation. In general, the above compositions may be prepared in a conventional manner using well known excipients, using standard techniques, including controlled release technologies, such as gelatin, lipid, gel depot, liposome and/or microcapsule based systems well known to those skilled in the art of pharmacy.
For an oral administration, the compounds of the invention will generally be provided in the form of tablets or capsule or as an aqueous solution or suspension.
Tablets for oral use may include the active ingredient mixed with pharmaceutically acceptable excipients such as inert diluents (such as sodium and calcium carbonate, sodium and calcium phosphate, or lactose), disintegrating agents (such as corn starch or alginic acid), binding agents (starch or gelatin), lubricating agents (magnesium stearate, stearic acid or talc), sweetening agents, flavoring agents, coloring agents and preservatives. If desired, the tablets may be coated with a material such as glyceryl monostearate or glyceryl distearate, to delay absorption in the gastrointestinal tract.
Capsules for oral use include hard gelatin capsules in which the active ingredient is mixed with a solid diluent, and soft gelatin capsules wherein the active ingredient is mixed with water or oil such as peanut oil, liquid paraffin or olive oil.
For intramuscular, intraperitoneal, subcutaneous and intravenous use, the compounds of the invention will generally be provided in sterile aqueous solutions (such as Ringer's solution or isotonic sodium chloride) or suspensions (such as cellulose derivatives, sodium alginate, polyvinylpyrrolidone and gum tragacanth with a wetting agent such as lecithin, and a preservative such as ethyl and n-propyl p-hydroxybenzoate), buffered to an appropriate pH and isotonicity.
Transdermal formulations include membrane permeation systems, multi-laminate adhesive dispersion systems and matrix dispersion systems. Transdermal delivery also includes the use of electrically aided transport and skin penetration enhancers.
The preferred route of administration is the intravenous infusion, preferably over a period of up to seven days, or as an oral formulation, or as an intramuscular injection via a styrette or as a subcutaneous injection.
It will be appreciated that the dosage levels used may vary over quite a wide range depending upon the compound used, the severity of the condition exhibited by the patient and the patient's body weight. However, without commitment to a rigid definition of dosages, it may be stated that a daily dosage of the active ingredient is 0,01 to about 100 mg/kg body weight of the patient being treated per day. Preferred dosages range from about 0,1 to about 10 mg/kg body weight/day.

Other characteristics and advantages of the invention are given in the following examples in which reference is made to:
- Figure 1 which represents the competition curve obtained with the compound described in example 1 at the human A₃ receptor;

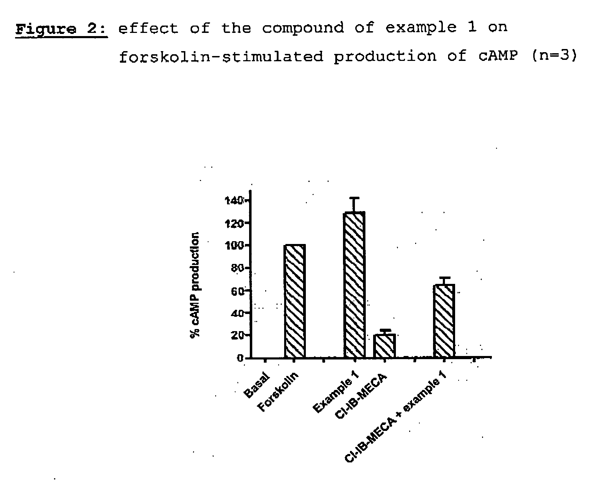

- Figure 2 which represents the effect of compound of the example 1 on forskolin-stimulated production of cAMP
It will be appreciated that the invention is described by way of example only and modification of detail may be made without departing from the scope of the invention.

### Examples:

### Preparation A: 3-Bromo-1-(2-nitro-phenyl)-1H-pyrrole-2-carboxylic acid methyl ester.

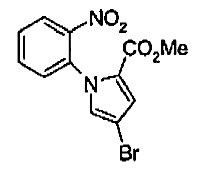

To a solution of methyl-4-bromo-pyrrole-2-carboxylate (5.0 g, 1.0 equiv) in DMF (45 mL) was added cesium carbonate (9.0 g, 1.2 equiv). The mixture was stirred at R.T. for 10 min, then 1-fluoro-2-nitrobenzene (3.9 mL, 1.5 equiv) was added. The reaction mixture was heated through microwave irradiation 5 min at 130°C. The reaction mixture was then diluted in AcOEt, washed with HCl 1M, water, brine and dried over MgSO₄. The organic layer was concentrated to give a dark brown oil. After triturating in Et₂O a solid was obtained and was filtered off, washed with Et₂O and dried to give the desired product as a yellow solid in 88% yield.

¹H-NMR (400 MHz, D6-DMSO): 3.59 (s, 3H, CH₃-O); 7.12 (d, *J* 1.9 Hz, 1H, Ar); 7.57 (d, *J* 1.9 Hz, 1H, Ar); 7.65 (dd, *J* 1.8 Hz, *J* 7.8 Hz, 1H, Ar); 7.76 (td, *J* 1.4 Hz, *J* 7.8 Hz, 1H, Ar); 7.87 (td, *J* 1.5 Hz, *J* 7.7 Hz, 1H, Ar); 8.21 (dd, *J* 1.5 Hz, *J* 8.1 Hz, 1H, Ar). M/Z (M+H)⁺= 327.

### Preparation B: 2-Bromo-5H-pyrrolo[1,2-a]quinoxalin-4-one.

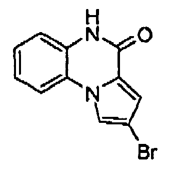

A suspension of compound from preparation A (6.4 g, 1 equiv) and iron powder (4.5 g, 4.0 equiv) in acetic acid (25 mL) was heated through microwave irradiation 8 min at 150°C. A dark solid was obtained and was suspended in HCl 1M, filtered off, washed with HCl 1M, AcOEt, Et₂O and dried to give a fluffy white solid in 90% yield.

¹H-NMR (400 MHz, D6-DMSO): 7.09 (d, *J* 1.5 Hz, 1H, Ar); 7.22 (t, *J* 7.0 Hz, 1H, Ar); 7.30 (m, 2H, Ar); 8.05 (d, *J* 8.0 Hz, 1H, Ar); 8.43 (d, *J* 1.6 Hz, 1H, Ar); 11.45 (bs, 1H, NH).
M/Z (M+14)¹= 265.

### Preparation C: 1-(2-Nitro-phenyl)-4-phenyl-1H-pyrrole-2-carboxylic acid methyl ester.

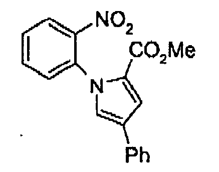

A mixture of compound from preparation A (2.50 g, 1 equiv), phenylboronic acid (1.87 g, 2 equiv), sodium carbonate (2.45 g, 3 equiv) and Pd(PPh₃)₄ (50 mg, 10%) in methanol/water (4:1) (25 mL) was heated through microwave irradiation 30 min at 150°C (Pₘₐₓ 70W). The reaction mixture was then diluted with AcOEt and washed with HCl 1M, water, brine and dried over MgSO₄. The organic layer was concentrated to give a brown oil. Purification by flash chromatography (5% to 15% AcOEt in cyclohexane) afforded a yellow solid that was triturated with Et₂O and dried to give the desired product in 22% yield.

¹H-NMR (400 MHz, D6-DMSO): 2.97 (s, 3H, CH₃-O) ; 7.23 (t, *J* 7.4 Hz, 1H, Ar); 7.37 (t, *J* 7.6 Hz, 2H, Ar); 7.50 (d, *J* 2.0 Hz, 1H, Ar); 7.70. (d, *J* 7. 9 Hz, 3H, Ar); 7.77 (td, *J* 1.4 Hz, *J* 7.7 Hz, 1H, Ar); 7.88 (m, 2H, Ar); 8.21 (dd, *J* 1.4 Hz, *J* 8.1 Hz, 1H, Ar).
M/Z (M+H)⁺ = 323.

### Preparation D: 3-(4-Methoxy-phenyl)-1-(2-nitro-phenyl)-1H-pyrrole-2-carboxylic acid methyl ester.

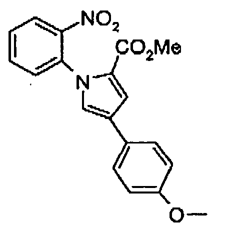

Compound obtained following the procedure described in preparation C from compound from preparation A and 4-methoxyphenylboronic acid in 54% yield as a yellow solid.

¹H-NMR (400 MHz, D6-DMSO): 3.61 (s, 3H, COOCH₃); 3.77 (s, 3H, CH₃O); 6.94 (dt, *J* 2.2 Hz, *J* 8.9 Hz, 2H, Ar); 7.43 (d, *J* 2.0 Hz, 1H, Ar); 7.62 (dt, *J* 2.2 Hz, *J* 8.9 Hz, 2H, Ar); 7.68 (dd, *J* 1.3 Hz, *J* 7.8 Hz, 1H, Ar); 7.73-7.78 (m, 2H, Ar); 7.88 (td, *J* 1.4 Hz, *J* 7.7 Hz, 1H, Ar); 8.20 (dd, *J* 1.4 Hz, *J* 8.1 Hz, 1H, Ar).
M/Z (M+H)⁺= 353.

### Preparation E : 4-Chloro-2-phenyl-pyrrolo[1,2-a]quinoxaline.

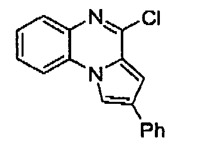

A suspension of compound from example 1 (500mg, 1 equiv) in phosphorus oxychloride (10 mL) was heated through microwave irradiation 10 min at 120°C. A yellow solid appeared and was filtered off, washed with CH₂Cl₂ and dried. The solid was then dissolved in DMF and reprecipitated by addition of water. The resulting white solid was filtered off, washed with Et₂O and dried to give the desired product in 36% yield.

¹H-NMR (400 MHz, D6-DMSO): 7.33 (t, *J* 7.2 Hz, 1H, Ar); 7.51 (m, 4H, Ar); 7.70 (t, *J* 7.8 Hz, 1H, Ar); 7.86 (dd, *J* 1.2 Hz, *J* 8.0 Hz, 1H, Ar); 7.92 (dd, *J* 1.2 Hz, *J* 8.2 Hz, 2H, Ar); 8.38 (dd, *J* 1.2 Hz, *J* 8.2 Hz, 1H, Ar); 9.16 (d, *J* 1.5 Hz, 1H, Ar).
M/Z (M+H)⁺ = 279.

### Preparation F: 4-Bromo-1-(5-fluoro-2-nitro-phenyl)-1H-pyrrole-2-carboxylic acid methyl 1 ester.

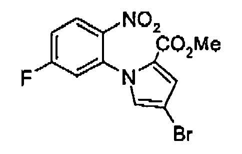

A suspension of 2,4-difluoronitrobenzene (448 µL, 1 equiv), methyl-4-bromopyrrole-2-carboxylate (1.0 g, 1.2 equiv) and cesium carbonate (1.2 g, 1 equiv) in DMF (10 mL) was heated through microwave irradiation 10 min at 100°C. The reaction mixture was then diluted in AcOEt, washed with HCl 1M, water, brine and dried over MgSO₄. The organic layer was concentrated to give a yellow oil. Purification by flash chromatography (5% to 20% AcOEt in cyclohexane) afforded a yellow solid in 30% yield.

¹H-NMR (400 MHz, D6-DMSO): 3.62 (s, 3H, COOCH₃); 7.13 (d, *J* 1. 9 Hz, 1H, Ar); 7.60 (d, *J* 1.9 Hz, 1H, Ar); 7.66 (m, 1H, Ar); 7.77 (dd, *J* 2.7 Hz, *J* 12.8 Hz, 1H, Ar); 8.33 (dd, *J* 5.4 Hz, *J* 9.2 Hz, 1H, Ar).
M/Z (M+H)⁺= 345.

### Preparation G: 4-Bromo-1-(5-dimethylamino-2-nitro-phenyl)-1H-pyrrole-2-carboxylic acid methyl ester.

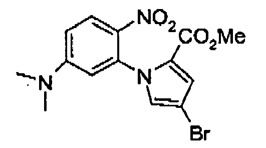

A mixture of compound from preparation F, diisopropylethylamine (50 µL, 1 equiv) and dimethylamine 2M solution in THF (1.5 mL) was heated through microwave irradiation 5 min at 100°C. The reaction mixture was then diluted in AcOEt, washed with HCl 1M, water, brine and dried over MgSO₄. The organic layer was concentrated to give a light orange solid in a quantitative yield.

¹H-NMR (400 MHz, D6-DMSO): 3.10 (s, 6H, N(CH₃)₂); 3.60 (s, 3H, COOCH₃); 6.67 (d, *J* 2.8 Hz, 1H, Ar); 6.84 (dd, *J* 2.8 Hz, *J* 9.5 Hz, 1H, Ar); 7.05 (d, *J* 1.9 Hz, 1H, Ar); 7.44 (d, *J* 1.9 Hz, 1H, Ar); 8.12 (d, *J* 9.5 Hz, 1H, Ar).
M/Z (M+H)⁺ = 368.

### Preparation H: 2-Bromo-8-dimethylamino-5H-pyrrolot 1,2-a]quinoxalin-4-one.

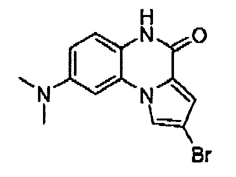

Compound obtained from compound from preparation G following the procedure described in preparation B in 80% yield as white needles.

¹H-NMR (400 MHz, D6-DMSO): 3.00 (s, 6H, N(CH₃)₂); 6.93 (b, 1H, Ar); 7.05 (d, *J* 1.7 Hz, 1H, Ar); 7.20 (d, *J* 8.9 Hz, 1H, Ar); 7.46 (b, 1H, Ar); 8.50 (d, *J* 1.8 Hz, 1H, Ar); 11.26 (bs, 1H, NH).
M/Z (M+H)⁺= 306.

### Preparation I: 4-Bromo-1-(5-morpholin-4-yl-2-nitro -phenyl)-1H-pyrrole-2-carboxylic acid methyl ester.

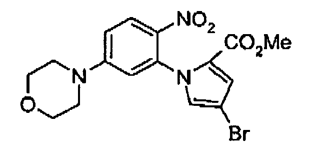

A mixture of compound from preparation F, morpholine (61 µL, 1.2 equiv) and diisopropylethylamine (122 µL, 1 equiv) in DMF (1 mL) was heated through microwave irradiation 5 min at 100°C. The reaction mixture was then diluted in AcOEt, washed with HCl 1M, water, brine and dried over MgSO₄. The organic layer was concentrated to give a yellow solid. Purification by flash chromatography (10% to 50% AcOEt in cyclohexane) afforded the desired product as a yellow solid in 80% yield.

¹H-NMR (400 MHz, D6-DMSO): 3.45 (m, 4H, N(CH₂)₂) ; 3.60 (s, 3H, COOCH₃); 3.71 (m, 4H, O(CH₂)₂) ; 6.99 (d, *J* 2.8 Hz, 1H, Ar); 7.07 (dd, *J* 2.8 Hz, *J* 9.5 Hz, 1H, Ar); 7.11 (d, *J* 2.8 Hz, 1H, Ar); 7.45 (d, *J* 2.0 Hz, 1H, Ar); 8.13 (d, *J* 9.5 Hz, 1H, Ar).
M/Z (M+H)⁺= 410.

### Preparation J: 1-(5-Morpholin-4-yl-2-nitro-phenyl)-4-phenyl-1H-pyrrole-2-carboxylic acid methyl ester.

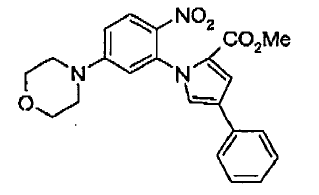

Compound obtained from compound from preparation I following procedure described in preparation C as a yellow solid in 60% yield.

¹H-NMR (400 MHz, D6-DMSO): 3.45 (m, 4H, N(CH₂)₂); 3.62 (s, 3H, COOCH₃); 3.72 (m, 4H, O(CH₂)₂) ; 7.02 (d, *J* 2. 7 Hz, 1H, Ar); 7.11 (dd, *J* 2.8 Hz, *J* 9.5 Hz, 1H, Ar); 7.21 (m, 1H, Ar); 7.36 (m, 2H, Ar); 7.45 (d, *J* 2.0 Hz, 1H, Ar); 7.68 (m, 2H, Ar); 7.75 (d, *J* 2.0 Hz, 1H, Ar); 8.14 (d, *J* 9.5 Hz, 1H, Ar).
M/Z (M+H)⁺ = 408.

### Preparation K: 4-(4-tert-Butoxycarbonylamino-phenyl)-1-(2-nitro-phenyl)-1H-pyrrole-2-carboxylic acid methyl ester.

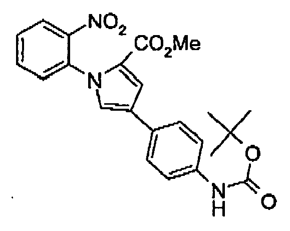

Compound obtained following the procedure described in preparation C from compound from preparation A and tert-butyl-N-[4-(4,4,5,5-tetramethyl-1,3,2-dioxaborolan-2-yl)phenyl]carbamatemethoxyphenylboronic acid in 44% yield as a yellow solid.

¹H-NMR (400 MHz, D6-DMSO): 1.49 (s, 9H, OOC(CH₃)₃); 3.61 (s, 3H, COOCH₃); 7.45 (m, 3H, Ar); 7.58 (m, 2H Ar); 7.68 (dd, *J* 1.4 Hz, *J* 7.8 Hz, 1H, Ar); 7.75 (m, 2H, Ar); 7.88 (td, *J* 1.5 Hz, *J* 7.7 .Hz, 1H, Ar); 8.20 (dd, *J* 1.4 Hz, *J* 8.1 Hz, 1H, Ar); 9.37 (bs, 1H, NH).
M/Z (M+H-C₄H₈)⁺ = 382.

### Preparation L: 4-(4-Dimethylaminopheny)-1-(2-nitrophenyl)-1H-pyrrole-2-carboxylic acid methyl ester.

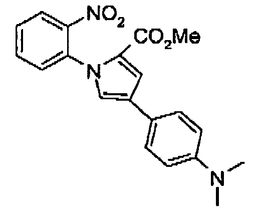

A mixture of compound from preparation K (150 mg, 1 equiv), formaldehyde 40% in aqueous solution (350 µL), sodium triacetoxyborohydride (210 mg, 3.2 equiv) and acetic acid (30 µL) in acetonitrile (1.5 mL) was stirred at R.T. After 2H, NaBH(OAc)₃ (105 mg) and formaldehyde (175 µL) were added and the reaction mixture was stirred for a further 2H. The reaction mixture was then diluted in AcOEt, washed with a saturated aqueous solution of NaHCO₃, brine, and dried over MgSO₄. The organic layer was concentrated to give an orange solid in a quantitative yield.

¹H-NMR (400 MHz, D6-DMSO): 2.90 (s, 6H, N(CH₃)₂); 3.30 (s, 3H, COOCH₃); 6.73 (m, 2H, Ar); 7.36 (d, *J* 2.0 Hz, 1H Ar); 7.50 (m, 2H, Ar); 7.66 (m, 2H, Ar); 7.14 (m, 1H, Ar); 7.87 (td, *J* 1.5 Hz, *J* 7.7 Hz, 1H, Ar); 8.19 (d, *J* 1.4 Hz, 1H, Ar).
M/Z (M+H)⁺= 366.

The following examples of compounds of formula (I) were obtained using above described routes of synthesis.

### Example 1: 2-Phenyl-5H-pyrrolo[1,2-a]quinoxalin-4-one.

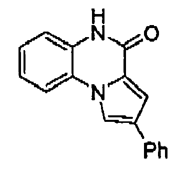

A mixture of compound from preparation B (500 mg, 1 equiv), phenylboronic acid (348 mg, 1.5 equiv), sodium carbonate (604 mg, 3 equiv) and Pd/C (2.5 mg, 0.5%) in methanol/water (4:1)(10 mL) was heated through microwave irradiation 10 min at 150°C (Pₘₐₓ 70W). A white solid was obtained, was filtered off and washed with water. The solid was then dissolved in DMF in order to remove the catalyst by filtration. Water was added to the filtrate to re-precipitate a white solid that was filtered off, washed with water, Et₂O and dried to afford the desired product in 80% yield.

Alternatively this compound can be obtained from compound from preparation C following the procedure described in preparation B in 61% yield as a fluffy white solid.

¹H-NMR (400 MHz, D6-DMSO): 7.22-7.34 (m, 4H, Ar); 7.40-7.48 (m, 3H, Ar); 7.82 (d, *J* 7.4 Hz, 2H, Ar); 8.12 (d, *J* 7.7, 1H, Ar); 8.72 (s, 1H, Ar); 11.32 (bs, 1H, NH).
M/Z (M+H)⁺= 261.
Mp = 301-303°C.

### Example 2: 2-(4-Methoxy-phenyl)-5H-pyrrolo[1,2-a]quinoxalin-4-one.

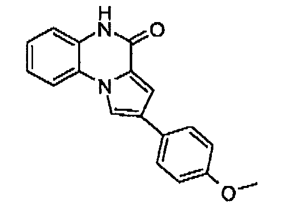

Compound obtained from compound from preparation D in 85% yield as a cream fluffy solid following the procedure described in preparation B.

¹H-NMR (400 MHz, D6-DMSO): 3.79 (s, 3H, OCH₃) ; 7.00 (d, *J* 8.7 Hz, 2H, Ar); 7.22-7.32 (m, 3H, Ar); 7.38 (d, *J* 1.6 Hz, 1H, Ar); 7.75 (d, *J* 8.8 Hz, 2H, Ar); 8.09 (d, *J* 7.9 Hz, 1H, Ar); 8.61 (d, *J* 1.6 Hz, 1H, Ar); 11.29 (bs, 1H, NH).
M/Z (M+H) ⁺ = 291.
Mp = 327-332°C.

### Example 3: Phenyl-(2-phenyl-pyrrolo[1,2-a]quinoxalin-4-yl)-amine.

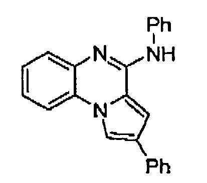

To a solution of compound from preparation E (60 mg, 1 equiv) in DMF (1 mL) was added aniline (39 µL, 2 equiv) and the mixture was heated through microwave irradiation 10 min at 150°C. The reaction mixture was then diluted in AcOEt, washed with water, brine and dried over MgSO₄. The organic layer was concentrated to give a yellow oil. Purification by flash chromatography (5% AcOEt in cyclohexane) afforded the desired product as a white solid in 55% yield.

¹H-NMR (400 MHz, D6-DMSO): 7.05 (t, *J* 7.3 Hz, 1H, Ar); 7.29-7.41 (m, 5H, Ar); 7.48 (m, 2H, Ar); 7.60-7.63 (m, 1H, Ar); 7.80-7.84 (m, 3H, Ar); 8.10 (dd, *J* 1.2 Hz, *J* 8.7 Hz, 2H, Ar); 8.17-8.20 (m, 1H, Ar); 8.86 (d, *J* 1.7, 1H, Ar); 9.18 (bs, 1H, NH).
M/Z (M+H)⁺= 336.
Mp = 172°C.

### Example 4 : Ethyl-(2-phenyl-pyrrolo[1,2-a]quinoxalin-4-yl)-amine hydrochloride.

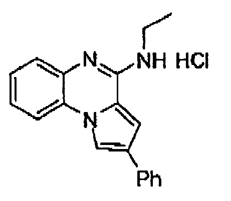

A suspension of compound from preparation E (70 mg, 1 equiv) in ethylamine 2M in THF (1 mL) was heated through microwave irradiation 2x 10 min at 150°C, 2x 5 min at 180°C and 10 min at 180°C. The reaction mixture was hydrolysed with HCl 1M and diluted with AcOEt. A white solid appeared and was filtered off, washed with water, AcOEt, Et₂O and dried to give the desired product as the corresponding hydrochloride salt in 37% yield.

¹H-NMR (400 MHz, D6-DMSO) : 1.36 (t, *J* 7.0 Hz, 3H, NCH₂CH₃); 3.82 (q, *J* 7.0 Hz, *J* 7.0 Hz, 2H, CH₃CH₂N); 7.34 (t, *J* 7*.*4 Hz, 1H, Ar); 7.16-7.52 (m, 4H, Ar); 7.78 (d, *J 7.2* Hz, 2H, Ar); 8.13 (bs, 1H, Ar); 8.29 (m, 2H, Ar); 9.07 (s, 1H, Ar); 10.21 (bs, 1H, NH); 12.56 (bs, 1H, NH).
M/Z (M+H)⁺= 288.
Mp = 299-301°C.

### Example 5: -Benzyl-(2-phenyl-pyrrolo[1,2-a]quinoxalin-4-yl)-amine.

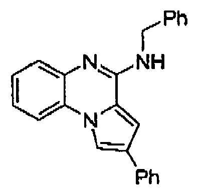

Compound obtained from compound from preparation E in 13% yield as a white solid following the procedure described in example 3.

¹H-NMR (400 MHz, D6-DMSO): 4.80 (d, *J 5.9* Hz, 2H, NCH₂Ph): 7.20-7.36 (m, 6H, Ar); 7.41-7.48 (m, 5H, Ar); 7.54 (d, *J* 1.7 Hz, 1H, Ar); 7.74-7.77 (m, 2H, Ar); 7.99 (t, *J* 5.7 Hz, *J 5. 9* Hz, 1H, Ar); 8.10 (dd, *J* 1.6 Hz, *J* 7.9 Hz, 1H, Ar); 8.74 (bd, *J* 1.6 Hz, 1H, NH).
M/Z (M+H)⁺ = 350.

### Example 6: Dimethyl-(2-phenyl-pyrrolo[1,2-a]quinoxalin-4-yl)-amine

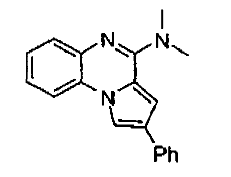

Isolated as a side product (white solid, 48% yield) during the purification of the compound described in example 5.

¹H-NMR (400 MHz, D6-DMSO) : 3.38 (s, 6H, (CH₃)₂N); 7.19-7.31 (m, 3H, Ar); 7.41-7.48 (m, 4H, Ar); 7.88 (m, 2H, Ar); 8.11 (dd, *J* 1.4 Hz, *J* 8.1 Hz, 1H, Ar); 8.80 (d, *J* 1.5 Hz, 1H, Ar).
M/Z (M+H)⁺ = 288.
Mp = 109-111°C.

### Example 7: Phenethyl-(2-phényl-pyrrolo[1,2-a]quinoxalin-4-yl)-amine.

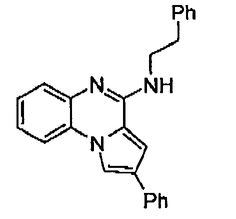

Compound obtained from compound from preparation E in 25% yield as a white solid following the procedure described in example 3.

¹H-NMR (400 MHz, D6-DMSO): 3.02 (t, *J* 7.8 Hz, 2H, NCH₂CH₂Ph); 3.76 (m, 2H, NCH₂CH₂Ph); 7.19-7.36 (m, 8H, Ar); 7.43-7.58 (m, 5H, Ar); 7.75 (dd, *J* 1.2 Hz, *J* 8.3 Hz, 2H, Ar); 8.09 (dd, *J* 1.4 Hz, *J* 8.0 Hz, 1H, Ar); 8.72 (bd, *J* 1.6 Hz, 1H, NH).
M/Z (M+H)⁺= 364.
Mp = 126°C.

### Example 8: 2-Phenyl-5H-pyrrolo[1,2-a]quinoxaline-4-thione

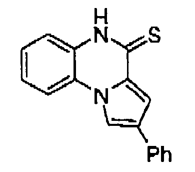

A suspension of compound from example 1 (100 mg, 1 equiv) and Lawesson's reagent (78 mg, 0.5 equiv) in a mixture of toluene (2.5 mL) and acetonitrile (1 mL) was heated through microwave irradiation 10 min at 130°C. A solid appeared and was filtered off, washed with Et₂O and dried to give a pale yellow solid in 76% yield.

¹H-NMR (400 MHz, D6-DMSO): 7.30 (t, *J* 7.4, 1H, Ar); 7.43 (m, 4H, Ar); 7.59 (m, 1H, Ar); 7.68 (d, *J* 1.7 Hz, 1H, Ar); 7.86 (dd, *J* 1.2 Hz, *J* 8.3 Hz, 2H, Ar); 8.21 (m, 1H, Ar); 8.90 (d, *J* 1.7 Hz, 1H, Ar); 12.99 (bs, 1H, NH).
M/Z (M+H)⁺ = 277.

### Example 9 : 4-Methylsulfanyl-2 phenyl pyrrolo[1,2-a]quinoxaline.

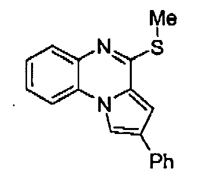

A solution of compound from example 8 (60 mg, 1 equiv) in THF (2 mL) was purged with N₂ and cooled to 0°C, then DBU (47 µL, 1.2 equiv) and methyl iodide (16 µL, 1.2 equiv) were added. The mixture was stirred at 0°C under N₂ for 1 hour, then the reaction mixture was diluted with AcOEt and washed with water, brine and dried over MgSO₄. The organic layer was concentrated to give a pale yellow solid. After trituration in a mixture of pentane and Et₂O a cream solid was isolated and dried to afford the desired product in 60% yield.

¹H-NMR (400 MHz, D6-DMSO) : 2.73 (s, 3H, CH₃-S) ; 7.30 (t, *J* 7.6 Hz, 1H, Ar); 7.33 (d, *J* 1.5 Hz, 1H, Ar); 7.42-7.50 (m, 3H, Ar); 7.56 (t, *J* 7.7 Hz, 1H, Ar); 7.82 (dd, *J* 1.3 Hz, *J* 7.3 Hz, 1H, Ar); 7.88-7.90 (m, 2H, Ar); 8.30 (dd, *J* 1.2 Hz, *J* 8.2 Hz, 1H, Ar); 9.97 (d, *J* 1.51 Hz, 1H, Ar).
M/Z (M+H)⁺= 291.
Mp = 132°C.

### Example 10: 2-(4-Dimethylamino-phenyl)-5H-pyrrolo[1,2-a]quinoxalin-4-one, trifluoroacetic acid salt.

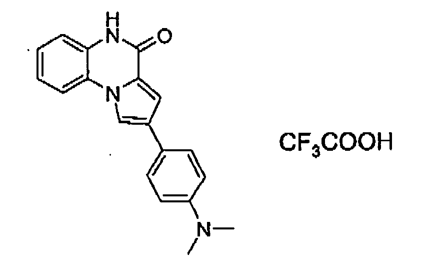

A suspension of compound from preparation L (120 mg, 1 equiv) and iron powder (440 mg, 2.2 equiv) in acetic acid (1 mL) was heated through microwave irradiation 10 min at 130°C. The reaction mixture was filtered off, and the filtrate was diluted with AcOEt. The organic layer was extracted with HCl 1M. The aqueous layer was basified with NaOH 6N and a solid precipitated. Purification of the solid by flash chromatography (20% to 100% AcOEt in cyclohexane) followed by purification by preparative chromatography afforded the product as a TFA salt.
M/Z (M+H)⁺= 304.

### Example 11: 8-Morpholin-4-yl-2-phenyl-5H-pyrrolo[1,2-a]quinoxalin-n-4-one, trifluoroacetic acid salt.

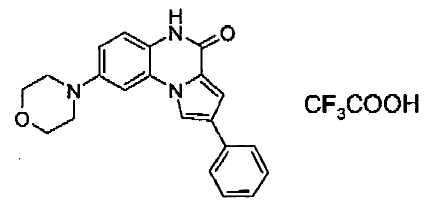

A suspension of compound from preparation J (110 mg, 1 equiv) and palladium 10% on carbon (10 mg) in a mixture of acetic acid (0.5 mL) and ethanol (5 mL) was hydrogenated overnight at R.T. under hydrogen atmosphere. The suspension was filtered off and washed with ethanol. The solid was then dissolved in hot DMF and filtered off. Water and NaHCO₃ were added to the filtrate, and the mixture was extracted with AcOEt. The organic layer was concentrated and was purified by preparative chromatography to give the product as a TFA salt.
M/Z (M+H)⁺ = 346.

### Example 12: Evaluation of the pharmacological activities of the compounds .

### Human A₁ receptor:

Compounds of the invention were evaluated in binding assays following procedure described by Townsend-Nicholson and Schofield (J. Biol. Chem. (1994), 269:2373-2376*) .* Human A₁ receptors were used in CHO cells with [³H]DPCPX (1nM) as radioligand, DPCPX (1µM) for non-specific binding and 60 minutes of incubation at 22°C.

### Human A_{2A} receptor:

Compounds of the invention were evaluated in binding assays following procedure described by Luthin et al. (Mol. Pharmacol. (1995), 47:307-313*).* Human A_{2A} receptors were used in HEK-293 cells with [³H]CGS 21680 (6nM) as radioligand, NECA (10µM) for non-specific binding and 90 minutes of incubation at 22°C.

### Human A_{2B} receptor:

Compounds of the invention were evaluated in binding assays following procedure described by Stehle et al. (Mol. Endocrinol. (1992), 6:384-393)*.* Human A_{2B} receptors were used in HEK-293 cells with [³H]MRS 1754 (0.5nM) as radioligand, NECA (1µM) for non-specific binding and 120 minutes of incubation at 22°C.

### Human A₃ receptor:

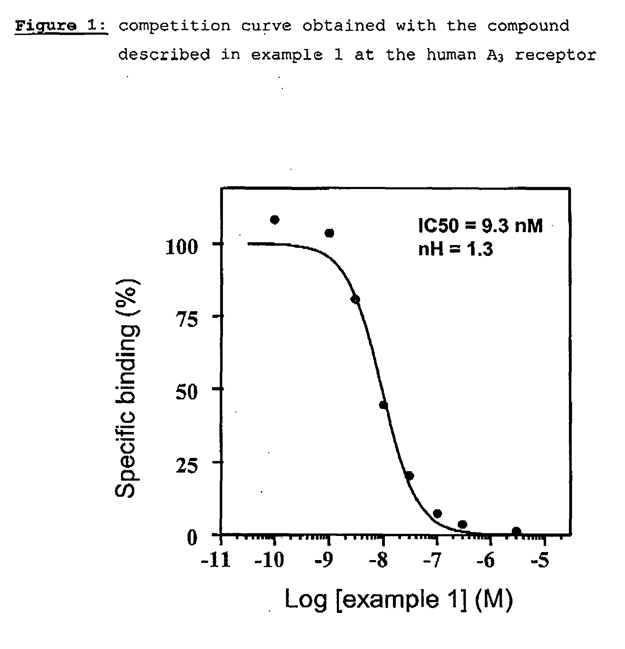

Compounds of the invention were evaluated in binding assays following procedure described by Salvatore et al. (Proc. Natl. Acad. Sci. (1993), 90:10365-10369)*.* Human A₃ receptors were used in HEK-293 cells with [¹²⁵I]AB-MECA (0.15nM) as radioligand, IB-MECA (1µM) for non-specific binding and 90 minutes of incubation at 22°C. Figure 1 shows competition curve obtained with compound of example 1 at the human A₃ receptor (hA₃).
The results for the compound of example 1 are summarized in the following table (I):

**Table (I)**

| | hA₁ IC₅₀ | hA_{2A} IC₅₀ | hA_{2B} IC₅₀ | hA₃ IC₅₀ |
|---|---|---|---|---|
| Example 1 | > 10 µM | > 10 µM | > 1 µM | 9.3 nM |
| Example 2 | ND | ND | ND | 82% inhibition at 1µM |

| | | | | |
|---|---|---|---|---|
| ND: not done | | | | |

### Functional assay on human A₃ receptor:

CHO cells expressing the human A₃ receptor were grown overnight as a monolayer in 24 well tissue culture plates (400 µl/well; 2x105 cells/well). cAMP generation was performed in Dulbecco's modified Eagle's medium (DMEM)/ *N-*2-hydroxyethylpiperazin-N-2-ethansulfonic acid (HEPES) buffer (0.60 g HEPES/50 ml DMEM pH 7.4). Each well was washed twice with HEPES/DMEM buffer (250 µl), and the following added: rolipram (50 µM) and cilostamide (50µM). This was incubated for 30 min at 37 °C followed by the introduction of the compound of example 1 (10 µM) and Cl-IB-MECA (0.1 µM) or DMEM/HEPES. After a further 10 min of incubation, forskolin was added (10 µM). After a subsequent 15 min, incubation was stopped by aspirating the assay medium and by adding 200 µl of ice-cold 0.1M HCl. The amount of cAMP was determined by competition with [3H]cAMP for protein kinase A (PKA). Briefly, the sample, approximately 1.8 nM [3H]cAMP, and 100µl of PKA solution were incubated on ice for at least 2.5h. The incubations were stopped by rapid dilution with 2 ml of icecold Tris HCl buffer (pH 7.4), and bound radioactive material was then recovered by filtration through Whatman GF/C filters. Filters were additionally rinsed with 2 x 2 ml of Tris HCl buffer, and then the radioactivity was counted in Packard Emulsifier Safe scintillation fluid (3.5 mL). All data reflect three independent experiments.

Effects of compound of example 1 on cAMP levels in CHO cells expressing the human A₃ receptor are summarized in the following table (II):

**Table (II)**

| | cAMP levels | | |
|---|---|---|---|
| | n=1 | n=2 | n=3 |
| Basal | 0.000 | 0.000 | 0.000 |
| Forskolin | 100.000 | 100.000 | 100.000 |
| Example 1 | 150.133 | 130.150 | 104.510 |
| 2-C1-IB-MECA | 23.252 | 23.252 | 14.006 |
| 2-Cl-IB-MECA + example 1 | 77.540 | 59.992 | 54.466 |

### Discussion:

Results show that compound of example 1 is a highly potent ligand for A₃ receptor. In addition, the compound of example 1 is selective for the subtype 3 for the adenosine receptor family as no detectable affinity was measured on A₁ nor A_{2A} up to 10µm and on A_{2B} up to 1µM. Compound of example 1 acts as an antagonist for A₃, receptor as it is able to prevent cAMP-decrease evoked by the reference agonist Cl-IB-MECA.
Compound of example 2 also shows potent affinity on A₃ receptor as it inhibits 82% of the specific binding at the concentration of 1µM.

### Conclusion:

Compound of example 1 is a potent and selective antagonist for the A₃ receptor.

## Claims

1. A compound of general formula (I): 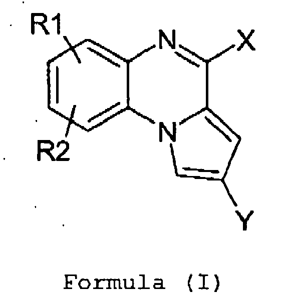 wherein:
**R1** and **R2** are independently from each other hydrogen, halogen, CN, CF₃, OCF₃, lower alkyl, COOH, O-(lower alkyl), S-(lower alkyl), N-(lower alkyl) (lower alkyl), heterocycloalkyl, aryl or heteroaryl;
**X** represents **R3** or CO**R3**, O-**R3** or S-**R3**, N-**R3R4**, NHCO**R3**, N(lower alkyl)CO**R3**, NHSO₂**R3**, N(lower **alkyl)SO₂R3** or NHCONH**R3** with
**R3** and **R4** are independently from the other hydrogen, alkyl, CF₃, aryl, heteroaryl, alkylaryl or alkylheteroaryl;
**Y** represents a **(A)ₙ-B** group wherein
**n** represents either 0 or 1,
**A** is a moiety selected in the group consisting of O, S, NH, N-(lower alkyl), N-aryl, N-heteroaryl,
**B** is an aryl or a heteroaryl group;
as well as pharmaceutically acceptable salt thereof, a prodrug of the compound or the salt, or a solvate or hydrate of the compound, the salt or the prodrug.

2. A compound according to claim 1, wherein **R1, R2** and **X** are as defined in claim 1 and Y represents an aryl group.

3. A compound according to claims 1 or 2, wherein **R1** is hydrogen, **R2** and **X** is as defined in claim 1 and **Y** represents an aryl group.

4. A compound according to any one of claims 1 to 3, wherein **R1** and **R2** are hydrogen, **X** is as defined in claim 1 and **Y** represents an aryl group.

5. A compound according to any one of claims 1 to 4, wherein **R1** and **R2** are hydrogen, **X** represents **R3,** O-**R3** or S-**R3**, N-**R3R4**, with:
**R3** and **R4** are independently from the other hydrogen, alkyl, CF₃, aryl, heteroaryl, alkylaryl or alkylheteroaryl;
and **Y** represents an aryl group.

6. A compound according to any one of claims 1 to 5 selected from *2-Phenyl-5H-pyrrolo[1,2-a]quinoxalin-4-one* and *2-(4-Methoxy-phenyl)-5H-pyrrolo[1,2-a]quinoxalin-4-one.*

7. A pharmaceutical composition comprising a pharmaceutically effective amount of a compound of formula (I) according to claim 1, or pharmaceutically acceptable salts thereof, in combination with a pharmaceutically acceptable carrier, diluent or excipient.

8. A method of modulating an A₃ receptor functioning by the administration of a therapeutically effective amount of a compound of general formula (I) according to claim 1 to a patient in need thereof, such that modulation of the adenosine receptor's activity occurs.

9. A method of selectively blocking an A₃ receptor by administration of a therapeutically effective amount of a compound of formula (I) to a patient in need thereof, such that blockage of the adenosine receptor's activity occurs.
